(19) Europäisches Patentamt
European Patent Office
Office européen des brevets

(11) Veröffentlichungsnummer: **0 001 223**
**A2**

(12) # EUROPÄISCHE PATENTANMELDUNG

(21) Anmeldenummer: 78100760.4

(22) Anmeldetag: 28.08.78

(51) Int. Cl.$^2$: G 01 N 33/16
C 08 G 81/02, C 08 B 37/02

(30) Priorität: 19.09.77 GB 38894/77

(43) Veröffentlichungstag der Anmeldung: 04.04.79 Patentblatt 79/7

(84) Benannte Vertragsstaaten: BE CH DE FR GB NL SE

(71) Anmelder: F.Hoffmann-La Roche & Co. Aktiengesellschaft
Abt. VIII-Pt
CH-4002 Basel(CH)

(72) Erfinder: Fischer, Ernst Alfred, Dr.
Rüttihardstrasse 2
CH-4142 Müchenstein(CH)

(74) Vertreter: Henrich, Werner et al,
Grenzacherstrasse 124 Postfach 601
CH-4002 Basel(CH)

(54) **Mit einer Polyhydroxyverbindung beschichteten Latex, Verfahren zur Herstellung dieses Latex, immunologisches Reagenz enthaltend diesen Latex, Verfahren zur Herstellung dieses Reagenzes, Verwendung dieses Reagenzes, Bestimmungsverfahren unter Verwendung dieses Reagenzes und Reagenziengarnitur enthaltend dieses Reagenz.**

(57) Es wird beschrieben ein Latex mit diskreten Teilchen eines Lates-Trägers, an den eine Polyhydroxyverbindung kovalent gebunden ist; ein Verfahren zur Herstellung dieses Latex; ein immunologisches Reagenz bestehend aus diesem Latex beschichtet mit einem immunologisch aktiven Material; ein Verfahren zur Herstellung dieses Reagenzes; die Verwendung dieses Reagenzes; ein Bestimmungsverfahren unter Verwendung dieses Reagenzes und eine Reagenziengarnitur enthaltend dieses Reagenz.

EP 0 001 223 A2

Croydon Printing Company Ltd.

RAN 4093/35

F. Hoffmann-La Roche & Co. Aktiengesellschaft, Basel/Schweiz

# Mit einer Polyhydroxyverbindung beschichteten Latex, Verfahren zur Herstellung dieses Latex, immunologisches Reagenz enthaltend diesen Latex, Verfahren zur Herstellung dieses Reagenzes, Verwendung dieses Reagenzes, Bestimmungsverfahren unter Verwendung dieses Reagenzes und Reagenziengarnitur enthaltend dieses Reagenz.

Die Erfindung bezieht sich auf ein neues Latex-Polymer und ein Verfahren zu seiner Herstellung. Sie bezieht sich ferner auf diagnostisch brauchbare Reagenzien, ein Verfahren zu deren Herstellung und solche Reagenzien verwendende Diagnosemethoden.

Die Diagnose pathologischer Zustände oder anderer Umstände im Menschen sowie bei Tieren erfolgt zunehmend unter Anwendung immunologischer Prinzipien, die zur Bestimmung des Vorhandenseins von Antikörpern oder Antigenen in einer Körperflüssigkeit des Versuchsobjekts angewandt werden.

Unter den verschiedenen bekannten Testmethoden, wie dem Agglutinationstest, dem Radioimmunoassay, dem Enzymimmunoassay, der Immunofluoreszenz oder der Immunodiffusion wird der Agglutinationstest als der billigste, einfachste und schnellste angesehen.

Hen/14.8.1978

Bei einem Agglutinationstest werden Träger verwendet, um die visuelle oder photometrische Unterscheidung der gebildeten Antigen-Antikörper-Komplexe, die von sehr geringer Größe sind, zu ermöglichen. Unter den Trägern, die verwendet worden sind, sind Schaf- und Human-Erythrozyten, Bakterienzellen, Bentonit, Latex-Teilchen, z.B. Polystyrol, oder carboxylierte Copolymerisate aus Styrol und Butadien, anionische Phenolharze und fein zerteilte diazotierte Aminocellulose.

Die bekannten Träger sind jedoch in ihrer Anwendbarkeit und Brauchbarkeit bei immunologisch-diagnostischen Arbeitsweisen beschränkt, da die entsprechenden immunologischen Reagenzien in vielen Fällen mangelnde Empfindlichkeit und/oder geringe Stabilität aufweisen.

Es war insbesondere nicht möglich, einen Agglutinationstest zur Bestimmung von Myoglobin im Urin von Patienten mit Verdacht auf Myokardinfarkt zu entwickeln, weil die bekannten immunologischen Träger beim Befestigen von Myoglobin unter Erhalt seiner immunologischen Eigenschaften nicht befriedigen oder nicht genügend Stabilität zeigen. Die bekannten Verfahren zur Bestimmung von Myoglobin im Urin, wie die Radioimmunodiffusion, sind teuer und mühselig. Insbesondere erlauben sie keine rasche Abschätzung des Myoglobins im Urin, die der Arzt am Bettrand des Patienten mit Verdacht auf Myokardinfarkt vornehmen könnte.

Es besteht also ein Bedürfnis nach einem Träger, der mit einem breiten Spektrum immunologisch aktiver Materialien, insbesondere mit Myoglobin, ein diagnostisch brauchbares Reagens bildet, das stabil, spezifisch, empfindlich ist und eine leicht festzustellende visuelle oder photometrische Auswertung in kürzester Zeit zuläßt.

Die Erfindung bezieht sich auf einen neuen Latex mit diskreten Teilchen eines Latex-Trägers, an den eine wasserlösliche Polyhydroxyverbindung kovalent gebunden ist.

Sie bezieht sich ferner auf ein Verfahren zur Herstellung eines solchen neuen Latex, bei dem eine wässrige Latex-Suspension mit einer wasserlöslichen Polyhydroxyverbindung umgesetzt wird.

Weiter bezieht sich die Erfindung auf einen aktivierten Latex, der durch Behandeln diskreter Teilchen eines Latex-Trägers hergestellt wird, an den eine wasserlösliche Polyhydroxyverbindung kovalent mit einem Aktivierungsmittel gebunden ist.

Die Erfindung bezieht sich weiter auf ein wasserunlösliches immunologisches Reagens mit einem spezifischen Gewicht von etwa dem von Wasser, das diskrete Teilchen eines Latex-Trägers aufweist, an den eine wasserlösliche Polyhydroxyverbindung kovalent gebunden ist, woran über eine kovalente Bindung ein bekanntes immunologisch aktives Material kondensiert ist.

Schließlich bezieht sich die Erfindung auf ein Verfahren zur Herstellung eines solchen wasserunlöslichen immunologischen Reagens, wobei ein immunologisch aktives Material mit einem aktivierten Latex, hergestellt durch Behandeln diskreter Teilchen eines Latex-Trägers, an den eine Polyhydroxyverbindung kovalent gebunden ist, mit einem Aktivierungsmittel, umgesetzt wird.

Der im Zusammenhang mit der Erfindung gebrauchte Ausdruck "Latex-Träger" oder "Kernpolymer" umfaßt Latex-Polymere, die wasserunlöslich sind, eine Teilchengröße im Bereich von etwa 0,01 bis etwa 0,9 µm, ein spezifisches Gewicht nahe

dem von Wasser haben, so daß nach dem Überziehen mit einer Polyhydroxyverbindung und Kuppeln mit dem immunologisch aktiven Material das spezifische Gewicht der Teilchen etwa 1,0 (0,95-1,05) beträgt, was sie in die Lage versetzt, ständig in wässriger Suspension zu verbleiben. Das Kernpolymer muß im Hinblick auf immunologisch-diagnostische Tests inert sein und aktive Gruppen aufweisen, die eine kovalente Bindung mit einer Polyhydroxyverbindung auszubilden vermögen. Beispielsweise können die Latex-Träger Carboxylgruppen, Amingruppen oder in diese überführbare Gruppen haben. Typische geeignete Gruppen an den Latex-Trägern sind solche, die einen aktiven Wasserstoff enthalten, z.B. $-COOH$, $-CONH_2$, eine Nitrilgruppe, eine primäre Amingruppe oder eine sekundäre Amingruppe.

Typische geeignete Latex-Teilchen sind solche, die als wässrige Latex-Suspension im Handel sind, gewöhnlich in Konzentrationen von etwa 40 bis etwa 60 % Feststoffen. Viele Arten Latex-Polymere sind zur erfindungsgemäßen Verwendung geeignet, vorausgesetzt, sie erfüllen die oben angeführten Kriterien. Die Erfindung umfaßt die Verwendung all dieser geeigneten Latices.

Bevorzugte Latexpolymere sind carboxylierte Latex-Polymere, wie carboxylierte Styrol-Butadiene, carboxylierte Polystyrole, carboxylierte Polystyrole mit Aminogruppen, Acrylsäure-Polymere, Methacrylsäure-Polymere, Acrylnitril-Polymere, Acrylnitril-Butadien-Styrole, Polyvinylacetat-Acrylate, Polyvinylpyridine, Vinylchlorid-Acrylate und dergleichen. Einige im Handel erhältliche Latices, die zur erfindungsgemäßen Verwendung geeignet sind, sind Amsco Res 4150, Amsco Res 3011 (American Mineral Spirits Co.); Dow Latex 815, Dow Latex 816, Dow Latex 620, Dow Latex 859, Dow Latex CL 241 (The Dow Chemical Co.); Hycar 1512, Hycar 1877X8, Hycar 2600x120 (Goodrich Chemical Co.); Gelva 900, Lytron 612, Lytron 624

(Monsanto); Rhoplex LC 3216, Amberlite Ultrafine (Röhm und Haas).

Besonders bevorzugte Latex-Polymere sind carboxylierte Styrol-Butadiene.

Der Begriff "Polyhydroxyverbindung" umfaßt alle Verbindungen mit wenigstens zwei Hydroxylgruppen, die die Latex-Oberfläche hydrophil zu machen vermögen und sich für eine kovalente Anbringung immunologisch aktiver Materialien eignen. Bevorzugte Polyhydroxyverbindungen sind wasserlösliche Polysaccharide oder deren Derivate, die Hydroxylgruppen und/oder primäre und/oder sekundäre Aminogruppen und/oder Carboxylgruppen enthalten. Beispiele für solche Polysaccharide sind Dextran, Agarose, wasserlösliche Cellulosederivate, wasserlösliche Stärke oder Dextrin. Besonders bevorzugt werden Polysaccharidderivate, wie Aminopolysaccharide, z.B. 1-Amino-2-hydroxypropyldextran oder p-Aminophenoxy-hydroxypropyldextran. Ferner können auch Copolymerisate von Polysacchariden oder anderen Hydroxyverbindungen mit bifunktionellen Substanzen, wie Copolymerisaten von Saccharose mit Zuckeralkoholen, wie Sorbit oder Mannit, verwendet werden.

Der Ausdruck "immunologisch aktives Material" bezieht sich auf Komponenten physiologischer Flüssigkeiten, Zell- und Gewebeextrakte , wofür ein immunologisches Gegenreagens zur Verfügung steht oder erzeugt werden kann. Typische immunologische Materialien sind primäre Amine, Aminosäuren, Peptide, Proteine, Lipoproteine, Glykoproteine, Sterine, Steroide, Lipoide, Nukleinsäuren, Enzyme, Hormone, Vitamine, Polysaccharide und Alkaloide.

Beispiele für solche immunologisch aktiven Substanzen sind in der folgenden Tabelle aufgeführt:

## Tabelle I

I. Mikroorganismen

Bakterien

1. Gram-positive Kokken
   Streptokokken (pyogenes, fecalis und viridans)
   Staphylokokken (aureus und albus)
   Pneumokokken (D. pneumoniae)
2. Gram-negative Kokken
   Neisseria (gonorrhoeae und meningitidis)
3. Gram-positive aerobe Bazillen
   Bacillus anthracis
   Corynebacterium diphtheriae
   Erysipelothrix
   Listeria monocytogenes
4. Gram-positive anaerobe Bazillen
   Clostridia (botulinum, perfringens, welchii und tetani)
5. Gram-negative anaerobe Bazillen
   Bacteroides
6. Gram-negative Intestinum-Bazillen
   Escherichia
   Klebsiella
   Enterobacter
   Proteus
   Pseudomonas
   Salmonella
   Shigella
7. Gram-negative nicht-intestinale Bazillen
   Pasteurella (pestis und tularensis)
   Hemophilus influenzae
   Brucella (melitensis, abortus und suis)

Bordetella pertussis
Malleomyces

8. Spirochäten
Treponema pallidum
Leptospira
Borrelia

9. Mycoplasma
10. Mycobacteria
11. Vibrio
12. Actinomyces

Protozoen

1. Intestinale Protozoen
Amöben

2. Flagellaten
Trichomonas
Leishmania
Trypanosomen
Toxoplasma

3. Sporozoen
Plasmodien (vivax, falciparum, malariae und ovale)

4. Intestinale Nematoden
Maden- oder Springwürmer
Hakenwürmer
Peitschenwürmer

5. Gewebe-Nematoden
Trichinella
Filaria (Wuchereria bancroftii)
Dracunculus

6. Trematoden
Schistosomen
Eingeweide-Saugwürmer
Gewebe-Egel

7. Cestoden
   Bandwürmer

8. Toxoplasma (T. gondii)

Fungi

1. Sporotrichum
2. Cryptococcus
3. Blastomyces
4. Histoplasma
5. Coccidioides
6. Candida

Viren und Rickettsien

1. Rickettsien

2. Viren
   Hunde-Hepatitis
   Shope-Papillome
   Influenza A & B
   Hühnerpest
   Herpes simplex
   Adenoviren
   Polyome
   Rous' Sarkom
   Impfpocken
   Poliovirus
   Masern
   Hundestaupe
   Leukämie
   Mumps
   Newcastle-Krankheit
   Sendai
   ECHO
   Maul- und Klauenseuche
   Psittacosis
   Tollwut
   Extromelia
   Baumviren

II. Gewebe-Antigene, einschließlich organspezifische Antigene

Polysaccharide
Hyaluronidase
Tetanus-Toxin
Ei-Ovalbumin
Ei-Serumalbumin

Niere
Leber
Haut
Herz (Myoglobin)
Magen-Darm-Trakt
Prostata
Embryo-Antigene (alpha 1 Fetoprotein)
Tumor-Antigene (Carcinoembryo-Antigen)
Muskel
Kollagen
Amyloid

III. <u>Hormone</u>

Hypophysen-Hormone
Insulin
Glucagon
Thyroid-Hormon
Choriongonadotropin
choriones Wachstumshormon - Prolactin
Human-Placenta-Lactogen

IV. <u>Enzyme</u>

Pankreas-Chymotrypsinogen
Procarboxypeptidase
Desoxyribonuclease
Ribonuclease
Glyceraldehyd-3-phosphat-dehydrogenase
Katalase
Peroxidase

V. <u>Blutzellen-Antigene, Blutgruppensubstanzen und andere Isoantigene</u>

Blutplättchen
Megakaryozyten
Leukozyten
Erythrozyten
Blutgruppensubstanzen
Forssmann-Antigen
Histokompatibilitäts-Antigene

VI. <u>Plasma-Proteine</u>

Fibrin und Fibrinogen
Plasminogen und Plasmin
Albumin
Immunoglobuline
$\alpha$-1-Antichymotrypsin
$\alpha$-1-Antitrypsin
Komplement-Faktoren
Ceruloplasmin
Gc-Globulin
Haptoglobin

α-2-Makroglobulin
ß-2-Mikroglobulin
Orosomucoid
Prealbumin
Transferrin

VII. Milch-Proteine

Lactoferrin
Lysozym
Sekret-IgA
Sekret-IgM
Sekret-Komponente

VIII. Speichel-Proteine

Sekret-IgA
Sekret-IgM
Sekret-Komponente

IX. Urin-Proteine

X. Pathologische Proteine

Myeloma-Protein
Makroglobulinaemische Proteine
Dysglobulinaemische Proteine
Bence Jones I, II-Proteine
C-reaktives Protein
Kryoglobuline

XI. Antikörper, einschließlich Autoantikörper

Antikernfaktor
Thyroid-Autoantikörper
Anti-Tamm-Horsfall-Protein
Kalt-Agglutinine
Rheumatoid-Faktor
Adrenal-Autoantikörper
Autoantikörper zu Magenwandzellen bei perniziöser Anämie
Anti-Colon
Anti-Leber
Anti-Niere
Autoantikörper zu Spermatozoen
Anti-Herz
Muskel-Autoantikörper bei Myasthenia gravis
Autoantikörper zu Nervengewebe
Autoantikörper gegen Fasergewebe und Gefäßkomponenten
Autoantikörper gegen Blutplättchen und Megakaryozyten
Antikörper gegen Trophoblasten
Antikörper zu Mikroorganismen
Antikörper zu tierischen Antigenen
Antikörper zu Arzneimitteln

Ein besonders bevorzugtes immunologisch aktives Material ist Myoglobin, Human-placentar-lactogen, Antikörper gegen IgG oder Antikörper gegen Myoglobin.

Der neue Latex gemäß der Erfindung kann durch Umsetzen einer wässrigen Latex-Suspension mit einer wasserlöslichen Polyhydroxyverbindung hergestellt werden.

Dies kann in herkömmlicher Weise in Abhängigkeit von den aktiven funktionellen Gruppen am Latex und der verwendeten wasserlöslichen Polyhydroxyverbindung erfolgen.

Bei einer bevorzugten Ausführungsform, bei der der verwendete Latex ein carboxyliertes Polymer und die wasserlösliche Polyhydroxyverbindung ein Aminopolysaccharid ist, verläuft die Reaktion, bei der eine Amidbindung ausgebildet wird, in Gegenwart eines Kupplungsmittels, wie eines wasserlöslichen Carbodiimids, des Woodward-Reagens K (N-Äthyl-5-phenyl-isoxazolium-3'-sulfonat) oder eines wasserlöslichen Chlorformiats.

Besonders bevorzugte Kupplungsmittel sind wasserlösliche Carbodiimide der Formel

$$R-N=C=N-R'$$

worin R oder R' Cycloalkyl mit 5 bis 6 Kohlenstoffatomen im Ring, Alkyl mit 2 bis 12 Kohlenstoffatomen, z.B. Äthyl, n-Propyl, Isopropyl, n-Butyl, sec.-Butyl, Isobutyl, tert.-Butyl, Amyl, Hexyl, Heptyl, Octyl, Nonyl, Decyl, Undecyl und Dodecyl, monoarylsubstituierte Niederalkylreste, z.B. Benzyl-, α- und ß-Phenyläthyl, Monoarylreste, z.B. Phenyl, Morpholino, Piperidyl, morpholinylsubstituierte Niederalkylreste, z.B. Äthylmorpholinyl, piperidylsubstituierte Niederalkylreste, z.B. Äthylpiperidyl, Diniederalkylamino, Niederalkylreste, pyridylsubstituierte Niederalkylreste, z.B. α-, ß- und γ-Methyl- oder -Äthylpyridyl sind, deren Säureadditionssalze und quaternären Amine.

Nach einer bevorzugten Ausführungsform der Erfindung werden das Aminopolysaccharid und eine wässrige Suspension des carboxylierten Kernlatex vorzugsweise bei Raumtemperatur (etwa 20 bis etwa 25°C) umgesetzt. Temperaturen von etwa 0 bis etwa 40°C sind jedoch für die Umsetzung auch geeignet. Um ein chemisches Kuppeln des Aminopolysaccharids an den Kernlatex zu gewährleisten, wird eine ausreichende Menge an Kupplungsmittel verwendet, um sicherzustellen, daß genügend Amidbindungen ausgebildet werden. Im allgemeinen sind etwa 0,005 bis etwa 6,0 Gewichtsprozent an wasserlöslichem Carbodiimid, bezogen auf das Gewicht der Teilchen, geeignet, gewöhnlich werden jedoch etwa 0,05 bis 2,0 Gewichtsprozent verwendet.

Der pH der Reaktion kann zwischen 2 und 7 variieren und liegt vorzugsweise bei 4 bis 5.

Die Reihenfolge der Zugabe des Aminopolysaccharids, des carboxylierten Kernlatex und des Kupplungsmittels ist unkritisch. Vorzugsweise wird jedoch in einer ersten Stufe das Kernlatex-Polymer mit dem wasserlöslichen Kupplungsmittel umgesetzt und in einer zweiten Stufe das Aminopolysaccharid zugefügt.

Das Verhältnis von Kernlatex zu Polyhydroxyverbindung in dem anfallenden Produkt kann innerhalb breiter Grenzen variieren und liegt vorzugsweise zwischen 2 und 50, wobei ein Verhältnis von 10 bis 30 besonders bevorzugt wird.

Das erfindungsgemäße neue Reagens kann durch Umsetzen des neuen Latex mit einem immunologisch aktiven Material hergestellt werden.

Dies kann in herkömmlicher Weise in Abhängigkeit von der wasserlöslichen Polyhydroxyverbindung und dem verwendeten

immunologisch aktiven Material geschehen. Bei einer bevorzugten Ausführungsform wird der neue Latex, der diskrete Teilchen eines Latex-Trägers aufweist, an den eine Polyhydroxyverbindung kovalent gebunden ist, zuerst aktiviert, vorzugsweise mit einem Oxydationsmittel, und dann mit dem immunologisch aktiven Material umgesetzt. Obgleich der aktivierte Latex gelagert werden kann, wird er vorzugsweise mit dem immunologisch aktiven Material unmittelbar nach der Aktivierung umgesetzt.

Bei einer besonders bevorzugten Ausführungsform, bei der die wasserlösliche Polyhydroxyverbindung ein Aminopolysaccharid ist, um später Vernetzungsprobleme zu vermeiden, werden alle Aminogruppen des Aminopolysaccharid-Latex, die an der Bindung an den Kern nicht beteiligt sind, in einer ersten Stufe durch Hydroxylgruppen ersetzt. Dies kann leicht durch Behandeln mit salpetriger Säure erfolgen. Der Polysaccharid-Latex wird dann mit Perjodat aktiviert, der einen Teil der Glucoseringe zu Dialdehyden oxydiert. Überschüssiges Perjodat wird mit Äthylenglykol umgesetzt. Nach dem Entfernen beider Reagenzien durch Waschen wird der so aktivierte Latex mit dem immunologisch aktiven Material umgesetzt. Zum Stabilisieren der durch die Reaktion der Aminogruppen des immunologisch aktiven Materials mit dem aktivierten Latex gebildeten Schiff-Basen und zur Beseitigung nicht-umgesetzter Aldehydgruppen wird das Produkt mit Natriumborhydrid mild behandelt. Dies reduziert die Schiff-Basen zu Aminen und Aldehydgruppen zu den entsprechenden Alkoholen, ohne die biologische Aktivität des immunologisch aktiven Materials wesentlich zu beeinflussen. Der Überschuß an immunologisch aktivem Material wird vorzugsweise, aber nicht notwendigerweise Reagenzien ausgesetzt und aus dem Reaktionsgemisch vor der Behandlung mit Natriumborhydrid entfernt. Der aktivierte Latex wird in der Kälte gewaschen, aber nach Zugabe des immunologisch aktiven Materials läßt man die Reaktion vorzugsweise bei Raumtemperatur ablaufen. Andererseits wird die Reduktion äußerst bequem in einem Eisbad bei $0^o$ durchgeführt, um die Denaturierung des immunologisch aktiven Materials minimal zu halten. Das Endprodukt wird mehrmals in Puffer gewaschen.

Die Menge an immunologisch aktivem Material, das an den neuen Latex gebunden ist, beträgt gewöhnlich etwa 0,01 bis 15,0 Gewichtsprozent. Jedes besondere immunologisch aktive Material wird jedoch in einer Menge verwendet, in der es bei einem Diagnosetest am erfolgreichsten eingesetzt wird. Daher wird jedes Material mit dem Träger in einem Verhältnis kombiniert, das für die speziellen Erfordernisse geeignet ist. Die Erfindung umfaßt daher die Verwendung einer Menge immunologisch aktiven Materials in Kombination mit einem immunologisch inerten Latex, die ausreicht, ein diagnostisch wirksames Reagens zu liefern.

Ist das Produkt einmal gebildet, kann es in spezifischen Diagnosetests unter Anwendung immunologischer Prizipien eingesetzt werden. Es kann in jeder passenden Konzentration in Abhängigkeit von dem speziellen Test verwendet werden, Konzentrationen von etwa 0,5 bis etwa 2,5 Gewichtsprozent sind jedoch geeignet, wobei 1,2 Gewichtsprozent bevorzugt werden. So kann beispielsweise das Produkt, das durch Kuppeln von Human-Myoglobin an den neuen Latex entsteht, als Diagnosereagens zur Bestimmung von Myoglobinurie bei einem Patienten mit Verdacht auf Myokardinfarkt verwendet werden. Dies kann z.B. durch Aufbringen eines Tropfens Testurin auf einen sauberen Glasträger, Mischen mit einem Tropfen geeignet verdünnten Antihuman-Myoglobinserums und anschließende Zugabe eines Tropfens des Myoglobin-Latex in wässriger Suspension erfolgen. Innerhalb einiger weniger Minuten sind die Testergebnisse zu beobachten, und zwar mit einer Genauigkeit von etwa 90-98 %.

Die Vorteile eines solchen Tests sind seine Einfachheit, Schnelligkeit, Spezifität, Genauigkeit und das Fehlen falscher positiver Angaben.

Als weiteres Beispiel kann das Produkt, das durch Kuppeln von Schaf-Antihuman-IgG an den neuen Latex entsteht, als Diagnosereagens zur Bestimmung von γ-Globulin in Serum oder anderen Körperflüssigkeiten verwendet werden.

Das erfindungsgemäße immunologische Reagens kann zur Bestimmung immunologisch aktiver Materialien bei einem direkten oder indirekten (Hemmung) Agglutinationstest oder bei der in der deutschen Patentanmeldung P 2749956 offenbarten kinetisch-photometrischen Methode verwendet werden.

Bei dem direkten Test werden die Probe und die Latex-Teilchen, die mit dem Gegenreagens für das zu bestimmende immunologisch aktive Material überzogen sind, gemischt und die Agglutination visuell oder photometrisch beobachtet. Der Test ist positiv, wenn Agglutination eintritt.

Beim indirekten Test wird die Probe mit dem Gegenreagens (z.B. Antiserum) für das zu bestimmende Material und dann mit Latex-Teilchen, die mit dem gleichen Material überzogen sind, gemischt. Die Agglutination wird visuell oder photometrisch beobachtet. Der Test ist positiv, wenn keine Agglutination eintritt.

Die erfindungsgemäßen immunologischen Reagenzien können für Handelszwecke bequem z.B. in einem Diagnosereagenssatz verpackt werden.

Für einen direkten Test enthält der Reagenssatz zur Bestimmung eines bekannten immunologisch aktiven Materials in einem Behälter ein wasserunlösliches immunologisches Reagens

mit einem spezifischen Gewicht von etwa dem von Wasser, das diskrete Teilchen eines Latex-Trägers aufweist, an den eine wasserlösliche Polyhydroxyverbindung kovalent gebunden ist, daran über eine kovalente Bindung ein immunologisches Gegenreagens für das zu bestimmende immunologisch aktive Material kondensiert.

Für einen indirekten Test enthält der Reagenssatz zur Bestimmung eines bekannten immunologisch aktiven Materials in einem ersten Behälter ein wasserunlösliches immunologisches Reagens mit einem spezifischen Gewicht von etwa dem von Wasser, das diskrete Teilchen eines Latex-Trägers aufweist, woran eine wasserlösliche Polyhydroxyverbindung kovalent gebunden ist, daran über eine kovalente Bindung das zu bestimmende immunologisch aktive Material ankondensiert, und in einem zweiten Behälter ein immunologisches Gegenreagens für das zu bestimmende immunologisch aktive Material.

Die folgenden Beispiele veranschaulichen die Erfindung.

## Beispiel 1

### Herstellung von 1-Amino-2-hydroxypropyl-dextran

Dieses lösliche Dextranderivat wurde aus der handelsüblichen Dextranfraktion T 70 mit einem durchschnittlichen Molekulargewicht von 70 000 (Pharmacia) hergestellt. 2 g Dextran T 70 wurden in 25 ml Wasser gelöst und 8,0 ml 1,0 n Natriumhydroxid zugesetzt. Zu dieser rasch gerührten Lösung wurden 7,05 g Epibromhydrin, in 12,5 ml Dioxan gelöst, bei 30°C gegeben und das Gemisch bei dieser Temperatur 4 h gehalten. Die Suspension wurde dann zur besseren Phasentrennung zentrifugiert und die untere Phase abpipettiert und verworfen. Die obere Phase wurde in einem Eisbad gekühlt, und 40 ml 25 %iger Ammoniaklösung sowie 10 ml 1,0 n Salzsäure wurden zugegeben. Dieses Reaktionsgemisch wurde bei Raumtemperatur in einem gasdichten Kolben 20 h gerührt. Dann wurde es bei

4°C gegen 12 l destilliertes Wasser mit 60 ml Eisessig dialysiert, worauf sieben Wechsel mit destilliertem Wasser folgten. Die dialysierte 1-Amino-2-hydroxypropyldextran-Lösung wurde auf ein Volumen von 34 ml durch Ultrafiltration eingeengt. Sie wurde durch Zusatz von 70 µl 20 %iger Natriumazidlösung stabilisiert und zur weiteren Verwendung im Kühlschrank aufbewahrt.

Herstellung von 1-Amino-2-hydroxypropyl-dextran T 70-überzogenem Latex

Carboxylierter Styrol-Butadien-Latex (Dow CL 241) wurde gewaschen und durch Behandeln mit dem Ionenaustauscherharz Dowex AG 50-X8, Bio Rad (Styrol-Divinylbenzol-Polymerlatex mit Sulfonsäuregruppen am Kern) in der Natriumform von Ammoniak befreit.

6 ml dieses Latex CL 241 in der $Na^+$-Form (Feststoffgehalt 78 mg/ml) wurde auf pH 4,75 eingestellt. Zu der rasch gerührten Suspension wurde eine Lösung aus 10 mg CMC (1-Cyclohexyl-3-[2-morpholinyl-(4)-äthyl]-carbodiimid-metho-p-toluolsulfonat) in 2 ml Wasser, unmittelbar vor Verwendung hergestellt, und nach 3 min 2 ml der 1-Amino-2-hydroxypropyl-dextran T 70-Lösung auf einmal zugegeben. Der pH wurde durch Zugabe von 0,1 n Natriumhydroxid auf 9,0 erhöht. Nachdem die Suspension über Nacht bei Raumtemperatur gerührt worden war, war der pH 8,4. Sie wurde mit Wasser auf etwa 40 ml verdünnt und 40 min bei 45 000 g zentrifugiert. Der Latex wurde dreimal mit etwa 40 ml Wasser, dann zweimal mit 0,1 m Natriumboratpuffer vom pH 8,5 und wieder zweimal mit Wasser gewaschen. Schließlich wurde der Bodensatz in 20 ml destilliertem Wasser suspendiert und 40 µl 20 %ige Natriumazidlösung wurde zur Stabilisierung zugesetzt. Aus vollständiger Hydrolyse einer Latex-Probe und einer Ninhydrin-Reaktion der Hydrolyseprodukte unter Vergleich mit dem Ausgangsmaterial wurde errechnet, daß 81 % des modifizierten

Dextrans an den Latex gebunden waren.

1,2-Dihydroxypropyl-dextran T 70-überzogener Latex

Dieses Derivat wurde durch Behandeln von 1-Amino-2-hydroxypropyl-dextran T 70-überzogenem Latex mit salpetriger Säure hergestellt: Zu 5 ml der fertigen Suspension des 1-Amino-2-hydroxypropyl-dextran T 70-überzogenen Latex wurden 300 µl 1,0 n Salzsäure unter Rühren und dann 100 µl 1,5 m Natriumnitrit in Wasser gegeben. Nach 5-minütiger Reaktion des Gemischs bei Raumtemperatur wurden 250 µl 1,0 n Natriumhydroxid zugesetzt. Die Suspension wurde mit Wasser auf etwa 12 ml verdünnt und 40 min bei 45 000 g zentrifugiert. Der Latex wurde zweimal mit etwa 12 ml Wasser gewaschen und dann in 5 ml Wasser aufgenommen. Zur Stabilisierung wurden 10 µl 20 %iges Natriumazid zugesetzt.

Herstellung von Myoglobin-dihydroxypropyldextran T 70-überzogenem Latex

35 ml Dihydroxypropyldextran T 70-überzogener Latex wurden in 7 ml 0,3 m Natriumbicarbonat suspendiert,und 7 ml 0,1 m Natriumperjodat in destilliertem Wasser wurden zugesetzt. Unter Lichtabschirmung wurde das Reaktionsgemisch bei Raumtemperatur 30 min gerührt. Dann wurden 7 ml 0,25 m Äthylenglykol zugesetzt und 1 h weitergerührt. Darauf wurde das Gemisch mit Wasser auf 40 ml verdünnt und 30 min bei 45 000 g (2°C) zentrifugiert. Die überstehende Flüssigkeit wurde dekantiert und der Latex einmal mit etwa 40 ml eiskaltem 0,01 m Natriumcarbonatpuffer vom pH 9,5 gewaschen, worauf wieder 30 min zentrifugiert wurde. Nun wurde der Bodensatz in 7 ml eiskaltem Carbonatpuffer aufgenommen.

Zu diesem aktivierten Latex wurde eine Lösung von 75 mg lyophilisiertem Myoglobin, in 3,5 ml Carbonatpuffer gelöst,

gegeben (dies entspricht 70 mg Myoglobin, wie spektrophotometrisch bestimmt wurde). Nach dem Einstellen des pH auf 9,5 mit 0,1 n Natriumhydroxid wurde die Suspension über Nacht bei Raumtemperatur gerührt (20 µl 20 %iges Natriumazid waren zur Stabilisierung zugesetzt worden). Dann wurde 40 min bei 45 000 g zentrifugiert. Die überstehende Flüssigkeit mit dem überschüssigen Myoglobin wurde abpipettiert und für weitere Versuche aufbewahrt. Das Pellet wurde in 7 ml eiskaltem Natriumcarbonatpuffer erneut suspendiert, und 14 ml frisch hergestelltes 0,2 %iges Natriumborhydrid in Carbonatpuffer wurde bei 0°C zugesetzt. Das Reaktionsgemisch wurde in einem Eisbad 5 h gerührt, dann zentrifugiert (45 min bei 45 000 g) und der Latex viermal mit etwa 40 ml 0,1 m Glycinpuffer vom pH 8,2 gewaschen. Schließlich wurde in 35 ml Glycinpuffer aufgenommen und mit 0,04 % Natriumazid stabilisiert. Aus dem Unterschied der optischen Dichte der Myoglobinlösung vor und nach der Kupplungsreaktion wurde berechnet, daß etwa 25 % des Proteins gekuppelt haben.

Röhrchentest

Der Röhrchentest wurde in Veronalacetatpuffer, pH 8,2, durchgeführt (48 mMol Natriumacetat + 48 mMol Natriumbarbital). 1,5 ml verdünntes Myoglobin-Antiserum (1:160) wurden mit 0,5 ml Urin gemischt, der verschiedene Mengen Myoglobin und 20 µl des Myoglobin-Latex enthielt. Urin mit 2,5 µg Myoglobin/ml hemmte die Agglutination für 90 min, während normale Urinproben in 40 min agglutinierten.

Objektträger-Test

Ein Tropfen Urin und ein Tropfen Antiserum wurden mit einem Kunststoffrührer gemischt. Dann wurde ein Tropfen Latex zugesetzt, die Probe wieder mit dem Rührer gemischt und gleichmäßig über den Kreis auf dem Objektträger ausgebreitet. Die

Stoppuhr wurde gestartet, während das Gemisch in kreisender Bewegung gehalten wurde. Nach 2 min wurde das Ergebnis abgelesen. Um eine Empfindlichkeit von 4 µg Myoglobin pro ml Urin zu erhalten, mußte das Antiserum auf 1:13,3 verdünnt werden, wozu der gleiche Veronalpuffer wie im Röhrchentest verwendet wurde. Die Latex-Suspension mußte mit Glycinpuffer auf 1:2,25 verdünnt werden.

## Beispiel 2

**Kuppeln von Schaf-Antihuman-IgG-Antikörpern an 1,2-Dihydroxypropyldextran T 70-überzogenen Latex CL 241**

5 ml desaminierter Latex, wie in Beispiel 1 beschrieben, wurden 30 min bei 45 000 g zentrifugiert. Die überstehende Flüssigkeit wurde dekantiert und der Latex in 1 ml 0,3 m Natriumbicarbonat suspendiert. 1 ml 0,1 m Natriumperjodat in destilliertem Wasser wurde zugesetzt, und das gegen Licht abgeschirmte Reaktionsgemisch wurde 30 min bei Raumtemperatur gerührt. Dann wurde 1 ml 0,25 m Äthylenglykol in Wasser zugesetzt und eine weitere Stunde gerührt. Das Gemisch wurde dann auf etwa 12 ml mit Wasser verdünnt und 30 min (bei $2^{o}C$) zentrifugiert. Die überstehende Flüssigkeit wurde dekantiert und der Latex wurde einmal mit 12 ml eiskaltem 0,01 m Natriumcarbonatpuffer, pH 9,5, gewaschen und wieder 30 min zentrifugiert. Der Bodensatz wurde nun in 0,5 ml eiskaltem 0,01 m Natriumcarbonatpuffer suspendiert, und 1 ml einer Schaf-Antihuman-IgG-Antikörper-Lösung im gleichen Puffer mit 5,8 mg Protein pro ml wurde zugegeben. Die Reaktion konnte bei $4^{o}$ über Nacht ablaufen. Die Suspension wurde dann zentrifugiert und die überstehende Flüssigkeit mit überschüssigem Antikörper abpipettiert. Der Bodensatz wurde in 1 ml eiskaltem Natriumcarbonatpuffer und 2 ml einer frisch hergestellten 0,2 %igen Natriumborhydridlösung in Carbonatpuffer wurde bei $0^{o}C$ zugesetzt. Nach 4-stündigem Rühren der Reduktionsmischung in einem Eisbad wurde sie zentrifugiert und die überstehende Flüssigkeit dekantiert. Der Latex wurde dann dreimal mit 12 ml 0,1 m Glycinpuffer, pH 8,2, gewaschen und mit 0,04 % Natriumazid stabilisiert. Schließlich wurde in 5 ml des gleichen Puffers aufgenommen.

Röhrchentest auf Human-IgG

Zu 2 ml Veronal-gepufferter Salzlösung (3,13 mMol Barbital, 1,82 mMol Natriumbarbital, 0,15 Mol Natriumchlorid, pH 7,5) mit 0,75 µg Human-IgG wurden 20 µl der obigen Latex-Suspension zugesetzt. Eine sichtbare Agglutination tritt innerhalb 75 min ein.

Objektträger-Test auf Human-IgG

60 µl Veronal-gepufferte Salzlösung mit 0,3 µg Human-IgG wird mit 20 µl der obigen Latex-Suspension gemischt. Spezifische Agglutination tritt innerhalb 30 s ein.

## Beispiel 3

Kuppeln von Human-placentar-lactogen (HPL) an 1,2-Dihydroxypropyldextran T 70-überzogenen Latex CL 241

5 ml desaminierter Latex, wie in Beispiel 1 beschrieben, wurde 30 min bei 45 000 g zentrifugiert. Die überstehende Flüssigkeit wurde dekantiert und der Latex in 1 ml 0,3 m Natriumbicarbonat suspendiert. 1 ml 0,1 m Natriumperjodat in destilliertem Wasser wurde zugesetzt, und das Reaktionsgemisch wurde unter Lichtabschirmung 30 min bei Raumtemperatur gerührt. Dann wurde 1 ml 0,25 m Äthylenglykol in Wasser zugesetzt und eine weitere Stunde gerührt. Das Gemisch wurde dann mit Wasser zu etwa 12 ml verdünnt und 30 min (bei 2$^{o}$C) zentrifugiert. Die überstehende Flüssigkeit wurde dekantiert und der Latex einmal mit 12 ml eiskaltem 0,01 m Natriumcarbonatpuffer, pH 9,5, gewaschen und wieder 30 min zentrifugiert. Der Bodensatz wurde nun in 0,5 ml eiskaltem

Carbonatpuffer suspendiert, und 1 ml einer 1 %igen Human-placentar-lactogen-Lösung in Wasser wurde zugesetzt. Die Reaktion konnte über Nacht bei Raumtemperatur ablaufen. Das Gemisch wurde dann 40 min zentrifugiert und die überstehende Flüssigkeit abpipettiert. Nach dem Suspendieren des Bodensatzes mit 1 ml Carbonatpuffer erfolgte die Reduktion bei $0^{o}C$ mit 2 ml einer frisch hergestellten 0,2 %igen Natriumborhydridlösung in dem gleichen Puffer. Dazu wurde das Reduktionsgemisch 4 h in einem Eisbad gerührt, dann mit Wasser auf etwa 12 ml verdünnt und 40 min zentrifugiert. Die überstehende Flüssigkeit wurde dekantiert und der Latex dreimal mit 0,1 m Glycinpuffer, pH 8,2, gewaschen. Schließlich wurde er in 5 ml des gleichen Puffers aufgenommen und mit 0,04 % Natriumazid stabilisiert.

Objektträger-Test auf HPL

60 µl der phosphatgepufferten Salzlösung (PBS) werden mit 20 µl Kaninchen-Anti-HPL-Serum, mit PBS 1:20 verdünnt, und 20 µl der obigen Latex-Suspension gemischt. Die Agglutination wird nach 1 min sichtbar.

Diese Agglutination kann durch Mischen von 20 µl Antiserum (in PBS 1:20 verdünnt) zuerst mit 30 µl PBS und 30 µl des gleichen Puffers mit 3 µg HPL spezifisch gehemmt werden. Mehr als 15 min zeigt sich kein Anzeichen einer Agglutination.

## Patentansprüche

1. Latex mit diskreten Teilchen eines Latex-Trägers, an den eine Polyhydroxyverbindung kovalent gebunden ist.

2. Latex nach Anspruch 1, dadurch gekennzeichnet, daß die Teilchen des Latex-Trägers eine Größe im Bereich von etwa 0,01 bis etwa 0,9 µm haben.

3. Latex nach Anspruch 1 oder 2, dadurch gekennzeichnet, daß das spezifische Gewicht der Teilchen des Latex-Trägers etwa 1,0 ist.

4. Latex nach einem der vorhergehenden Ansprüche, dadurch gekennzeichnet, daß der Latex-Träger ein carboxylierter Latex ist.

5. Latex nach Anspruch 4, dadurch gekennzeichnet, daß der carboxylierte Latex ein carboxyliertes Styrol-Butadien-Copolymerisat ist.

6. Latex nach einem der vorhergehenden Ansprüche, dadurch gekennzeichnet, daß die wasserlösliche Polyhydroxyverbindung ein wasserlösliches Polysaccharid oder ein Derivat hiervon ist.

7. Latex nach Anspruch 6, dadurch gekennzeichnet, daß das wasserlösliche Polysaccharid ein Aminopolysaccharid ist.

8. Latex nach Anspruch 7, dadurch gekennzeichnet, daß das Aminopolysaccharid 1-Amino-2-hydroxypropyldextran ist.

9. Latex nach einem der vorhergehenden Ansprüche, dadurch gekennzeichnet, daß das Verhältnis von Träger-Latex zur wasserlöslichen Polyhydroxyverbindung zwischen 2 und 50 liegt.

10. Latex nach Anspruch 9, dadurch gekennzeichnet, daß das Verhältnis von Träger-Latex zur wasserlöslichen Polyhydroxyverbindung zwischen 10 und 30 liegt.

11. Latex nach einem der vorhergehenden Ansprüche in durch Behandeln mit einem Aktivierungsmittel erzielter aktivierter Form.

12. Latex nach Anspruch 11, dadurch gekennzeichnet, daß er mit einem oxydierenden Mittel aktiviert wurde.

13. Latex nach Anspruch 12, dadurch gekennzeichnet, daß er mit einem Perjodat als oxydierendem Mittel aktiviert wurde.

14. Wasserunlösliches, immunologisches Reagens mit einem spezifischen Gewicht von etwa dem von Wasser, mit diskreten Teilchen eines Latex-Trägers, an den eine wasserlösliche Polyhydroxyverbindung kovalent gebunden

ist, woran über eine kovalente Bindung ein bekanntes immunologisch aktives Material kondensiert ist.

15. Reagens nach Anspruch 14, dadurch gekennzeichnet, daß die Teilchen des Latex-Trägers eine Größe im Bereich von etwa 0,01 bis etwa 0,9 µm aufweisen.

16. Reagens nach Anspruch 14 oder 15, dadurch gekennzeichnet, daß der Latex-Träger ein carboxylierter Latex ist.

17. Reagens nach Anspruch 16, dadurch gekennzeichnet, daß der carboxylierte Latex ein carboxyliertes Styrol-Butadien-Copolymerisat ist.

18. Reagens nach einem der Ansprüche 14 bis 17, dadurch gekennzeichnet, daß die wasserlösliche Polyhydroxyverbindung ein wasserlösliches Polysaccharid oder ein Derivat hiervon ist.

19. Reagens nach Anspruch 18, dadurch gekennzeichnet, daß das wasserlösliche Polysaccharid ein Aminopolysaccharid ist.

20. Reagens nach Anspruch 19, dadurch gekennzeichnet, daß das Aminopolysaccharid 1-Amino-2-hydroxypropyldextran ist.

21. Reagens nach einem der Ansprüche 14 bis 20, dadurch gekennzeichnet, daß das Verhältnis von Träger-Latex zu der wasserlöslichen Polyhydroxyverbindung zwischen 2 und 50 liegt.

22. Reagens nach Anspruch 21, dadurch gekennzeichnet, daß das Verhältnis von Träger-Latex zu der wasserlöslichen Polyhydroxyverbindung zwischen 10 und 30 liegt.

23. Reagens nach einem der Ansprüche 14 bis 22, dadurch gekennzeichnet, daß das immunologisch aktive Material Myoglobin ist.

24. Reagens nach einem der Ansprüche 14 bis 22, dadurch gekennzeichnet, daß das immunologisch aktive Material Human-placentar-lactogen ist.

25. Reagens nach einem der Ansprüche 14 bis 22, dadurch gekennzeichnet, daß das immunologisch aktive Material ein Antikörper gegen IgG ist.

26. 1-Amino-2-hydroxypropyldextran.

27. Verfahren zur Herstellung eines Latex gemäß einem der Ansprüche 1 bis 10, dadurch gekennzeichnet, daß eine wäßrige Latex-Suspension mit einer wasserlöslichen Polyhydroxyverbindung umgesetzt wird.

28. Verfahren nach Anspruch 27 zur Herstellung eines Latex, dessen Träger-Latex ein carboxyliertes Polymer und dessen wasserlösliche Polyhydroxyverbindung ein Aminopolysaccharid ist, dadurch gekennzeichnet, daß die wässrige Latex-Suspension mit dem Aminopolysaccharid in Gegenwart eines Kupplungsmittels umgesetzt wird.

29. Verfahren nach Anspruch 28, dadurch gekennzeichnet, daß als Kupplungsmittel ein wasserlösliches Carbodiimid verwendet wird.

30. Verfahren zur Herstellung eines aktivierten Latex nach einem der Ansprüche 11 bis 13, dadurch gekennzeichnet, daß ein Latex gemäß einem der Ansprüche 1 bis 10 mit einem aktivierenden Mittel behandelt wird.

31. Verfahren nach Anspruch 30, dadurch gekennzeichnet, daß als aktivierendes Mittel ein oxydierendes Mittel verwendet wird.

32. Verfahren nach Anspruch 31, dadurch gekennzeichnet, daß das oxydierende Mittel ein Perjodat ist.

33. Verfahren zur Herstellung eines Reagens gemäß einem der Ansprüche 14 bis 25, dadurch gekennzeichnet, daß ein immunologisch aktives Material mit einem aktivierten Latex, hergestellt durch Behandeln diskreter Teilchen eines Latex-Trägers, an den eine Polyhydroxyverbindung kovalent gebunden ist mit einem aktivierenden Mittel, umgesetzt wird.

34. Verfahren zum Bestimmen eines immunologisch aktiven Materials in einer Probe, bei dem die Probe mit einem wasserunlöslichen immunologischen Reagens mit einem spezifischen Gewicht von etwa dem von Wasser, mit diskreten Teilchen eines Latex-Trägers, an den eine wasserlösliche Polyhydroxyverbindung kovalent gebunden ist, daran über eine kovalente Bindung ein immunologisches Gegenreagens für das zu bestimmende immunologisch aktive Material ankondensiert, in Berührung gebracht und das Eintreten einer Agglutination beobachtet wird.

35. Verfahren nach Anspruch 34, dadurch gekennzeichnet, daß als zu bestimmendes immunologisch aktives Material -Globulin und als immunologisches Gegenreagens Schaf-Antihuman-IgG verwendet wird.

36. Verfahren zum Bestimmen eines immunologisch aktiven Materials in einer Probe, bei dem die Probe mit einem immunologischen Gegenreagens für das zu bestimmende immunologisch aktive Material und mit einem wasserunlöslichen immunologischen Reagens mit einem spezifischen Gewicht von etwa dem von Wasser, mit diskreten Teilchen eines Latex-Trägers, an den eine wasserlösliche Polyhydroxyverbindung kovalent gebunden ist, daran über eine kovalente Bindung das zu bestimmende immunologisch aktive Material ankondensiert, in Berührung gebracht und das Eintreten einer Agglutination beobachtet wird.

37. Verfahren nach Anspruch 35, dadurch gekennzeichnet, daß als zu bestimmendes immunologisch aktives Material Myoglobin und als immunologisches Gegenreagens Myoglobin-Antiserum verwendet wird.

38. Reagenssatz für die Bestimmung eines bekannten immunologisch aktiven Materials, enthaltend in einem Behälter ein wasserunlösliches immunologisches Reagens mit einem spezifischen Gewicht von etwa dem von Wasser, das diskrete Teilchen eines Latex-Trägers aufweist, an den eine wasserlösliche Polyhydroxyverbindung kovalent gebunden ist, daran über eine kovalente Bindung ein immunologisches Gegenreagens für das zu bestimmende immunologisch aktive Material ankondensiert.

39. Reagenssatz nach Anspruch 38, dessen zu bestimmendes immunologisch aktives Material $\gamma$-Globulin und dessen immunologisches Gegenreagens Schaf-Antihuman-IgG ist.

40. Reagenssatz zur Bestimmung eines bekannten immunologisch aktiven Materials, enthaltend in einem ersten Behälter ein wasserunlösliches immunologisches Reagens mit einem spezifischen Gewicht von etwa dem von Wasser, das diskrete Teilchen eines Latex-Trägers aufweist, an den eine wasserlösliche Polyhydroxyverbindung kovalent gebunden ist, daran über eine kovalente Bindung das zu bestimmende

immunologisch aktive Material ankondensiert, und in einem zweiten Behälter ein immunologisches Gegenreagens für das zu bestimmende immunologisch aktive Material.

41. Reagenssatz nach Anspruch 40, dadurch gekennzeichnet, daß das zu bestimmende immunologisch aktive Material Myoglobin und das immunologische Gegenreagens Myoglobin-Antiserum ist.

42. Verwendung eines Latex gemäß einem der Ansprüche 1 bis 10 als Träger bei immunologischen Bestimmungen.

43. Verwendung eines aktivierten Latex gemäß einem der Ansprüche 11 bis 13 als Träger bei immunologischen Bestimmungen.

44. Verwendung eines Reagens nach einem der Ansprüche 14 bis 25 bei immunologischen Bestimmungen.

***